(19) **Europäisches Patentamt European Patent Office Office européen des brevets**

(11) **EP 2 061 583 B1**

(12) **EUROPEAN PATENT SPECIFICATION**

| | |
|---|---|
| (45) Date of publication and mention of the grant of the patent:<br>**10.04.2019 Bulletin 2019/15** | (51) Int Cl.:<br>**B01J 3/02** *(2006.01)* **A61K 9/16** *(2006.01)* |
| (21) Application number: **07805595.1** | (86) International application number:<br>**PCT/IL2007/001136** |
| (22) Date of filing: **16.09.2007** | (87) International publication number:<br>**WO 2008/032327 (20.03.2008 Gazette 2008/12)** |

(54) **ORGANIC NANOPARTICLES OBTAINED FROM MICROEMULSIONS BY SOLVENT EVAPORATION**

ORGANISCHE NANOPARTIKEL, ERHALTEN AUS MIKROEMULSIONEN DURCH LÖSUNGSMITTELVERDAMPFUNG

NANOPARTICULES ORGANIQUES OBTENUES À PARTIR DE MICROÉMULSIONS PAR ÉVAPORATION DE SOLVANT

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR** | (72) Inventors:<br>• **MAGDASSI, Shlomo**<br> **96626 Jerusalem (IL)**<br>• **NETIVI, Hadas**<br> **38497 Hadera (IL)**<br>• **GOSHEN, Katrin**<br> **96625 Jerusalem (IL)** |
| (30) Priority: **14.09.2006 US 844365 P** | |
| (43) Date of publication of application:<br>**27.05.2009 Bulletin 2009/22** | (74) Representative: **Becker Kurig Straus**<br>**Patentanwälte**<br>**Bavariastrasse 7**<br>**80336 München (DE)** |
| (73) Proprietor: **Yissum Research Development Company,**<br>**of The Hebrew University of Jerusalem**<br>**91390 Jerusalem (IL)** | (56) References cited:<br>**WO-A-2005/072709    WO-A-2005/102507**<br>**US-A- 5 472 706** |

EP 2 061 583 B1

**Description**

**FIELD OF THE INVENTION**

The present invention relates to nanoparticles of a water-insoluble organic compound in the form of a redispersible powder, and a process for the production of such nanoparticles from microemulsions.

**BACKGROUND OF THE INVENTION**

Many drug compounds, skin treatment materials and food preservatives are substantially insoluble in water. There are several different approaches to solve the solubility problem of poorly water-soluble compounds. These include traditional solubilizing approaches using a combination of solvents, surfactants and co-solvents, various dispersions techniques, as well as micronization, complexation and liposomal delivery techniques.

One approach directed to delivery and release of poorly soluble drugs includes their formulation as nano-sized particles (nanoparticles). Nanoparticles of organic compounds can be produced through the use of microemulsions, comprising either water-in-oil "reverse" microemulsions, or oil-in-water microemulsions. Nanoparticles prepared from water-in-oil microemulsions have been disclosed with respect to each of cholesterol, Rhovanil and Rhodiarome (Debuigne et al. Langmuir 2000, 16(20), 7605-7611), and nimesulide (Debuigne et al. J. Pharm Belg. 2000 55(2), 59-60).

Nanoparticles prepared by solvent diffusion (not solvent evaporation) technique using oil-in-water microemulsion have been disclosed with respect to griseofulvin, an antifungal drug (Trotta M., et al. Int. J. Pharm. 2003, 245, 235-242). Latex nanoparticles can also be obtained by polymerization (not solvent evaporation) in oil-in-water microemulsion (Ozer et al. J. App. Poly. Sci. 2000, 78(3), 569-575). Solid lipid microspheres can be prepared by solidification (not solvent evaporation) from oil-in-water microemulsion (U.S. Patent No. 5,250,236). Finally, pigment nanoparticles can be produced from oil-in-water microemulsion in ink-jet printing process by evaporation of volatile solvent on substrate surface. (Magdassi and Ben Moshe Langmuir 2003, 19(3), 939-942).

Nanoparticles obtained using emulsion (not microemulsion) and solvent evaporation technique have been disclosed with respect to cellulose derivatives and polylactic acid (Desgouilles et al. Langmuir 2003, 19, 9504-9510); and pilocarpine encapsulated within poly(lactide-co-glycolide) polymer (Yoncheva et al. J. Microencapsul. 2003, 20(4), 449-458).

WO 2005/072709 discloses a drug delivery system comprising nanoparticles of a water poorly soluble drug dispersed in a polymeric hydrophilic bead, and a method for producing the drug delivery system. The disclosed method comprises mixing an oil-in-water submicron emulsion comprising a poorly water soluble drug, with a water-soluble bead forming polymer; providing conditions enabling bead formation; optionally evaporating volatile organic solvent and water used in earlier steps, and thereby obtaining dry beads containing dispersed nanoparticles of the poorly water soluble drug.

WO 2005/102507 describes a process for preparing nanoparticles from oil-in-water nanoemulsions, in which the nanoemulsion is prepared by phase inversion, or temperature inversion techniques.

WO 2005/020933 relates to a process for the preparation of polymeric nanoparticles with target molecules bonded to the surface of the particles and having sizes of up to 1000nm, preferably 1nm to 400nm, more preferably 1nm to 200nm that are dispersed homogeneously in aqueous solution. The polymeric nanoparticles are prepared using emulsion polymerization technique.

WO 01/88046 describes a process to pattern organic nanoparticles by ink-jet printing of microemulsions.

U.S. Pat. No. 5,879,715 relates to a process for the production of inorganic nanoparticles by precipitating the inorganic nanoparticles by a precipitating agent from a microemulsion with a continuous and a non-continuous phase and concentrating the precipitated nanoparticles employing an ultra filtration membrane.

U.S. Pat. No. 5,874,029 describes the production of microparticles and nanoparticles in which a compressed fluid and a solution including a solvent and a solute are introduced into a nozzle to produce a mixture. The mixture is then passed out of the nozzle to produce a spray of atomized droplets. The atomized droplets are contacted with a supercritical antisolvent to cause depletion of the solvent in the droplets so that the particles are produced from the solute. Preferably, these particles have an average diameter of 0.6 μm or less. This process relies on a spraying type vaporization process.

US 2005/0170004 relates to nanoparticles comprising an organic wax and a surfactant, wherein a peptide, polysaccharide or glycoprotein is electrostatically attached to the nanoparticle. Hsu et al. (AAPS PharmSciTech 2003, 4(3), E32) relates to nanoparticles of encapsulated coenzyme Q10 in lyophilized and aqueous suspension forms, and their production from microemulsions.

US 5,472,706 and US 5,750,142 disclose a lyophilized composition comprising submicron particles and an amino compound cryoprotectant, and a method of making a lyophilized composition, wherein the method involves use of a submicron oil-in-water emulsion comprising an amino compound cryoprotectant. European Patent No. 211257 relates to lyophilized emulsion compositions comprising carbohydrates which are intended for parenteral administration of hydrophobic drugs.

As outlined above, prior art nanoparticle formulations intended for delivery of water-insoluble organic compounds comprise encapsulating materials and/or special ingredients and techniques required for maintaining the final material in powder or dispersed form. Such formulations and methods suffer from increased production time and costs, and more complicated regulatory approval processes.

There remains an unmet need for nanoparticle formulations of water-insoluble organic compounds which can be produced by simple and cost-effective techniques.

## SUMMARY OF THE INVENTION

The present invention provides redispersible powders comprising nanoparticles of water-insoluble organic compounds and methods for their production. The invention is based, in part, on the unexpected discovery that a redispersible powder formulation or an aqueous dispersion, comprising high weight percentages of a water-insoluble organic compound in the form of nanoparticles, can be prepared from an oil-in-water microemulsion or nanoemulsion containing a water insoluble, volatile organic solvent, from which organic solvent and/or water has been removed.

The simultaneous removal of the water and the organic solvents, for example by spray drying, leads to immediate conversion of the dissolved organic material, into solid nanoparticles. These nanoparticles exhibit surprising properties in view of their dissolution properties, and degree of non-crystallinity.

Advantageously, the redispersable powder provides a product having a long shelf life, with enhanced dissolution properties, and can be converted to an aqueous dispersion, if desired. The nanoparticle formulations advantageously comprise a high percentage by weight of the water-insoluble organic compound in particulate form.

In a first aspect, the present invention provides a redispersible powder comprising nanoparticles of a water-insoluble organic compound, the nanoparticles having a diameter of less than 30 nanometers and comprising at least 50% by weight of the water-insoluble organic compound, wherein the redispersible powder is prepared by a process comprising the steps of: (i) preparing an oil-in-water microemulsion comprising a water-insoluble organic compound, a volatile water-immiscible organic solvent, water, and at least one surfactant, by: (a) dissolving the water-insoluble organic compound in the volatile water-immiscible organic solvent so as to form an organic phase ; and (b) mixing the organic phase with water and at least one surfactant so as to spontaneously form the oil-in-water microemulsion without the use of a high pressure homogenizer or a high shear instrument; wherein the diameter of the microemulsion droplets is below 30nm; and (ii) removing the volatile water-immiscible organic solvent and the water so as to form the redispersible powder comprising said nanoparticles, wherein said nanoparticles are in a particulate form, wherein the volatile water-immiscible organic solvent and the water are removed simultaneously.

As used herein, the term "particulate form" means individual non-aggregated particles, excluding, e.g., nanoparticles dispersed in a matrix such as a polymeric bead.

Additional constituents of the redispersible powder such as polymers, (preferably non-cross linked polymers) and redispersion aids are present in the powder as separate entities and not as a part of the particles of the water-insoluble organic compound. Without wishing to be limited by any particular mechanism or theory, it is believed that the role of the polymers is to improve stability of the nanoparticles, to enable control of the dissolution rate, to prevent crystallization, and in addition, in the case of water soluble polymer, to aid the re-dispersion process. Suitable polymers include, but are not limited to, water insoluble polymers such as polylactic acid, cellulose acetate, methyl cellulose, hydroxylpropyl methyl cellulose, poly(lactic-co-glycolic acid), hydroxylpropyl cellulose phthalate, and mixtures thereof. Alternatively, the polymer can be a water soluble polymer such as polyvinyl pyrrolidone (PVP), polyvinyl alcohol, carboxy methyl cellulose, hydroxy ethyl cellulose, polyethylene glycol, gum arabic and mixtures thereof. Alternatively, the polymer can be a non-crosslinked polymer. Examples of a re-dispersion aid include, but are not limited to, a wetting agent, a disintegrant, a water soluble polymer, colloidal silica particles, sugars, mannitol and mixtures thereof.

The nanoparticles may comprise at least about 80% by weight, more preferably 85%, still more preferably 90% yet still more preferably 95% by weight of the water-insoluble organic compound. The nanoparticles may consist essentially of the water-insoluble organic compound. The nanoparticles may comprise at least about 5% of the redispersible powder.

Advantageously, preparation of the water-insoluble organic compound in nanoparticulate form significantly increases its solubility and rate of dissolution as compared to the same drug in unprocessed form, i.e., in a form which has not undergone any particle size reduction or other treatment to increase its solubility and rate of dissolution. Thus, the solubility of the water-insoluble organic compound can be at least about 5 times greater than the solubility of the water-insoluble organic compound in unprocessed form-i.e. not in the form of the nanoparticles prepared by the invention. The solubility of the water-insoluble organic compound can be at least about 10 times greater than the solubility of the water-insoluble organic compound in unprocessed form. The dissolution rate of the nanoparticles can be at least about 5 times greater than the dissolution rate of the water-insoluble organic compound in unprocessed form, or the dissolution rate of the nanoparticles can be at least about 10 times greater than the dissolution rate of the water-insoluble organic compound in unprocessed form.

In another embodiment, the water-insoluble organic compound is in an amorphous form or a partially amorphous form. An aqueous dispersion may comprise the redispersible powder of the invention, and an aqueous medium.

Disclosed but not forming part of the present invention is a pharmaceutical composition comprising the redispersible powder of the invention, and at least one inert excipient. The powder may be packed within a capsule or a tablet or a granule. The pharmaceutical composition can be in the form of an aqueous dispersion, and may comprise the redispersible powder of the invention dispersed in an aqueous medium.

In another aspect, the present invention provides a process for preparing a redispersible powder comprising nanoparticles of a water-insoluble organic compound, the nanoparticles having a diameter of less than 30 nanometers and comprising at least 50% by weight of the water-insoluble organic compound, the process comprising the steps of: (i) preparing an oil-in-water microemulsion comprising a water-insoluble organic compound, a volatile water-immiscible organic solvent, water, and at least one surfactant, by: (a) dissolving the water-insoluble organic compound in the volatile water-immiscible organic solvent so as to form an organic phase ; and (b) mixing the organic phase with water and at least one surfactant so as to spontaneously form the oil-in-water microemulsion without the use of a high pressure homogenizer or a high shear instrument; wherein the diameter of the microemulsion droplets is below 30nm; and (ii) removing the volatile water-immiscible organic solvent and the water so as to form the redispersible powder comprising said nanoparticles, wherein said nanoparticles are in a particulate form, wherein the volatile water-immiscible organic solvent and the water are removed simultaneously , wherein the nanoparticles are in a particulate form.

The step of preparing the microemulsion does not include the use of a high pressure homogenizer or a high sheer instrument. In another embodiment, the volatile water-immiscible organic solvent and the water are removed by spray drying or lyophilization. In another embodiment, the process for preparing a redispersible powder further comprises the step of re-dispersing the powder in water to form an aqueous dispersion of the nanoparticles.

The water-immiscible organic solvent may be selected from the group consisting of n-butyl acetate, sec-butyl acetate, isobutyl acetate, propyl acetate, toluene, xylenes, R(+)-limonene, hexane, pentane, heptane and mixtures thereof. The water-insoluble organic compound may be present in an amount of about 0.1 to about 20% by weight, the water-immiscible organic solvent may be present in an amount of about 0.5 to about 50% by weight, the co-solvent may be present from 0 to about 30% and the water may be present in an amount of from about 20 to about 85% by weight, based on the total weight of the microemulsion.

In another embodiment, the microemulsion further comprises at least one polymer. In other embodiments, the polymer is a water insoluble polymer selected from polylactic acid, cellulose acetate, methyl cellulose, hydroxylpropyl methyl cellulose, poly(lactic-co-glycolic acid), hydroxylpropyl cellulose phthalate, and mixtures thereof. In other embodiments, the polymer is a water soluble polymer selected from the group consisting of polyvinyl pyrrolidone (PVP), polyvinyl alcohol, carboxy methyl cellulose, hydroxy ethyl cellulose, polyethylene glycol, gum arabic and mixtures thereof. In another embodiment, the polymer is a non-crosslinked polymer. In another embodiment, the polymer is present in an amount of about 0.01 to about 10% by weight based on the total weight of the microemulsion.

In another embodiment, the microemulsion further comprises a re-dispersion aid, Examples of a re-dispersion aid include, but are not limited to, a wetting agent, a disintegrant, a water soluble polymer, colloidal silica particles, sugars, mannitol and mixtures thereof.

In another embodiment, the microemulsion further comprises a co-solvent. Examples of a co-solvent include, but are not limited to, ethanol, 1-propanol, 2-propanol, n-pentanol, n-butanol, ethyl acetate, tetrahydrofuran, propylene glycol, formamide, glycerol, polyethylene glycol and mixtures thereof. The co-solvent may be present in an amount of about 5 to about 30% by weight based on the total weight of the microemulsion.

In another embodiment, the surfactant is selected from the group consisting of a cationic surfactant, an anionic surfactant, an amphoteric surfactant, a nonionic surfactant and mixtures thereof. In specific embodiments, the anionic surfactant is selected from the group consisting of an alkyl benzene sulphonate, sodium dodecyl sulfate, sodium sulfosuccinate, sodium lauryl sulfate, an alkyl naphthalene sulfonate condensate sodium salt, sodium stearate, and mixtures thereof; the nonionic surfactant is selected from the group consisting of an ethoxylated sorbitan ester, a sorbitan ester, a polyglycerol ester, a sucrose ester, a poloxamer, an alkyl polyglucoside, a polyalkyleneoxide modified heptamethyltrisiloxane, an allyloxypolyethylene glycol methylether and mixtures thereof; the amphoteric surfactant is lecithin; and the cationic surfactant is selected from the group consisting of cetyl trimethyl ammonium bromide, cetyl trimethyl ammonium chloride, and mixtures thereof. The surfactant can be present in an amount of about 5 to about 35% by weight based on the total weight of the microemulsion.

In another embodiment, the process further comprises the step of crystallizing the nanoparticles, thereby providing crystalline organic nanoparticles. In another embodiment, the nanoparticles are crystallized by aging.

Further disclosed but not forming part of the present invention is a process for preparing an aqueous dispersion comprising nanoparticles of a water-insoluble organic compound, the process comprising the steps of: i) preparing an oil-in-water microemulsion comprising a water-insoluble organic compound, a polymer, a volatile water-immiscible organic solvent, water, and at least one surfactant; and (ii) removing the volatile water-immiscible organic solvent so as to form an aqueous dispersion comprising the nanoparticles, wherein the nanoparticles are in a particulate form.

In specific embodiments, the water-insoluble organic compound is selected from the group consisting of pharmaceutically active agents, cosmetic active agents, anti oxidants, preservatives, colorants, food additives, agriculturally active compounds (e.g., pesticides, herbicides and fertilizers), reagents used in chemical and biochemical reactions such as water-insoluble peptides, and fragrances. The anti oxidant may be selected from the group consisting of butylated hydroxytoluene (BHT), butylated hydroxy anisol (BHA) and carnosic acid; and the preservative may be selected from the group consisting of methyl paraben, ethyl paraben, propyl paraben, and butyl paraben.

Further embodiments and the full scope of applicability of the present invention will become apparent from the detailed description given hereinafter. However, it should be understood that the detailed description and specific examples, while indicating preferred embodiments of the invention, are given by way of illustration only.

## BRIEF DESCRIPTION OF THE FIGURES

Figure 1 is an x-ray diffractogram of a physical mixture containing simvastatin, Tween® 80, soybean lecithin, hydrochloric acid and sucrose prior to formulation as nanoparticles.

Figure 2 is an x-ray diffractogram of crystalline simvastatin (raw material) prior to formulation as nanoparticles.

Figure 3 is an x-ray diffractogram of a simvastatin nanoparticle preparation containing: simvastatin, Tween® 80, soybean lecithin, hydrochloric acid and sucrose.

Figure 4 is a CryoTEM image of a simvastatin nanoparticle preparation containing: simvastatin, Tween® 80, soybean lecithin, hydrochloric acid and sucrose.

## DETAILED DESCRIPTION OF THE INVENTION

The invention relates, in one embodiment, to a redispersible powder comprising nanoparticles of a water-insoluble organic compound, wherein the nanoparticles are in a particulate form, according to claim 12. A redispersible powder provides a product having a long shelf life and possessing minimal bulk and weight properties (as compared to a liquid form). The nanoparticles have a better dissolution rate and better solubility then the conventional microparticles, and this may lead to enhanced bioavailability, for example, for poorly soluble drugs. If desired, a redispersible powder can be converted to an aqueous dispersion upon contact with an aqueous medium such as water. Also disclosed is an aqueous dispersion comprising nanoparticles of a water-insoluble organic compound, wherein the nanoparticles are in a particulate form. Such redispersible powders and aqueous dispersions can be used for a variety of applications, including, for example, delivery and administration of pharmaceutically active compounds, incorporation of food processing materials such as preservatives and anti oxidants into processed food products, incorporation of cosmetic additives into cosmetic products, delivery of agriculturally active compounds such as poorly water soluble pesticides, and delivery of reagents used in chemical and biochemical reactions such as water-insoluble peptides. The invention further relates to processes for preparing such redispersible powders, wherein the processes comprise preparation of an oil-in-water microemulsion and subsequent solvent removal.

Definitions

The term "nanoparticles" as used herein describes particles having an average diameter of between about 1 nanometer (nm) and about 1000 nm. Nanoparticles of a particular molecular entity or compound exhibit physico-chemical properties that are significantly different from that of larger forms of the same molecular entity or compound. The nanoparticles have a diameter of less than 30 nm.

Solubility is defined as the concentration of the solute in a saturated solution. The solubility of compounds varies in accordance with factors such as temperature, the type of solvent, the pH of the solution, and atmospheric pressure. Further, decreased size leads to increased solubility and dissolution rate. As described in a review by Sasson et al. (in Insecticide Design Using Advanced Technologies, edited by: Isaac Ishaaya, Ralf Nauen and Rami Horowitz, Springer-Verlag, Heidelberg, Germany), downsizing of a drug particle, particularly to the submicron level, leads to simultaneous enhancement of both the saturation solubility $C_s$ and the dissolution rate dC/dt. The saturation solubility increases with decreasing particle size according to the Ostwald-Freundlich Equation, also known as the Gibbs-Thomson Equation and as the Kelvin Equation (Equation 1):

<u>Equation 1:</u>

$$\frac{S\,(d)}{S_0} = \exp \frac{\gamma\,V_m}{RTd}$$

where S(d) is the solubility (mol/kg $H_2O$) of crystals with inscribed diameter d (m) at temperature T (°K), $V_m$ is the molar volume ($m^3$/mol), $\gamma$ is the surface free energy (surface tension) (mJ/$m^2$); R is the gas constant (8314.5 mJ/mol.°K); and $S_0$ is the solubility of the bulk material (d→∞). With all other factors kept constant the solubility increases with smaller particle size. However for the solubility S(d) to differ significantly from the solubility $S_0$ of the bulk material (i.e. the ratio S(d)/$S_0$ >> 1) the exponential term needs to be much smaller than one. This occurs only with particle size in the nanometric range.

In addition, the dissolution rate (dC/dt) is directly proportional to the surface area and to the concentration gradient. This is determined by the Noyes-Whitney Equation (Equation 2):

<u>Equation 2:</u>

$$\frac{dC}{dt} = \frac{DA(C_S - C_B)}{h}$$

where C is concentration (mole/liter), D is the diffusion coefficient of the drug, h is the effective diffusion boundary layer, A is the effective surface area, $C_S$ is the saturation solubility of the drug (equivalent to S in Equation 1) and $C_B$ is the bulk concentration of the drug. Since upon decrease of the particles into the nanometric range, both $C_s$ and A increase, the effect on the dissolution rate is significant.

The solubility of compounds is expressed as the number of milliliters of solvent in which one gram of solute can dissolve. Where the exact solubility of various compounds cannot be precisely determined general quality terms are used to describe the solubility of a specific compound, typically with reference to other compounds. Solubility may also be expressed in terms of molality, percentage, and morality. Typically, compounds defined as water insoluble are those that require more than 1 ml part of solvent per 10 mg of solute (1% w/v).

The term "in a particulate form" as used herein denotes discrete, individual, non-aggregated particle entities composed of a water-insoluble organic compound, such that the water-insoluble organic compound is not enclosed within, incorporated within, embedded within, contained within or associated with any encapsulation form, bead, carrier, matrix or similar delivery agent.

The term "dissolution factor" as used herein describes the relative dissolution rate of a solute in a solvent. In particular, the term describes the relative time required to dissolve specific proportions of a solvent and a solute which are required in order to effect dissolution of the solute in the solvent. In addition it describes the increase in maximal solubility compared to the maximal solubility of the bulk material.

The term "microemulsion" as used herein includes both oil-in-water microemulsions and "reverse" microemulsions which are water-in-oil microemulsions. An oil-in-water microemulsion is a translucent to transparent dispersion of an organic phase in an aqueous phase, having a droplet diameter size in the nanometer range (1-50 nm). It is thermodynamically stable and is generally spontaneously self emulsifying upon mixture of appropriate surfactant(s), cosurfactant(s), solvent(s), cosolvent(s), water insoluble material and water (see for example, Friberg et al. (1987) Microemulsions Structure and Dynamics, CRC Press Inc., Boca Raton, Fla.). In contrast, oil-in-water emulsions having droplets of larger diameter can be thermodynamically unstable and/or require high shear forces to induce their formation. A "reverse microemulsion" is a water-in-oil microemulsion which is a translucent to transparent dispersion of an aqueous phase in an organic phase and it is also thermodynamically stable

The oil-in-water microemulsion is a dispersion or emulsion of droplets of a water-insoluble, volatile organic solvent in an aqueous medium, with the droplets having an oily core surrounded by an interfacial film of at least one surfactant. The surfactant(s) function in emulsifying the water-insoluble organic compound, wherein the emulsification process denotes the formation of the droplets dispersed within the aqueous phase. The droplets contain dissolved organic, water insoluble material, defined as the "active material".

In one embodiment, the present invention provides a method for preparing a redispersible powder comprising nanoparticles of a water-insoluble organic compound by preparing an oil-in-water microemulsion, followed by removing the liquid components i.e. the volatile water immiscible organic solvent and the water so as to form the redispersible powder, as specified in claim 1. The water-insoluble organic solvent and the water are preferably removed simultaneously, but can also be removed sequentially, in any order. The microemulsion used in this method comprises a water-insoluble organic compound, a volatile water immiscible organic solvent, water and at least one surfactant. The microemulsion can further comprise additional components, in particular, at least one polymer, a re-dispersion aid or a co-solvent. Advantageously, the redispersible powder can be further dispersed in an aqueous medium e.g., water, to produce an aqueous dispersion.

Also disclosed is a method for preparing an aqueous dispersion of a water-insoluble organic compound by, preparing an oil-in-water microemulsion, followed by removing the volatile water immiscible organic solvent so as to form the aqueous dispersion. A polymer is preferably added to the microemulsion. Thus, the microemulsion may comprise a water-insoluble organic compound, a volatile water-immiscible organic solvent, a polymer, water, and at least one surfactant. The second step of this method involves removal of the volatile water-immiscible organic solvent but not the water, so that an aqueous medium remains. The microemulsion can further comprise a co-solvent.

As used herein, a water-insoluble organic compound refers to an organic compound which is insoluble or poorly soluble in water. According to the invention, the water-insoluble organic compound can be a pharmaceutically active agent, a cosmetic active agent, an anti oxidant, a preservative, a colorant, a food additive, an agriculturally active compound such as a pesticide, or a herbicide, a reagent used in chemical and biochemical reactions such as water-insoluble peptides, or a fragrance.

A pharmaceutically active agent refers to a chemical or biological molecule having therapeutic, diagnostic or prophylactic effects *in vivo.* Contemplated pharmaceutically active agents for use in the invention described herein include general anesthetics, local anesthetics, hypnotics, sedatives and anxiolytics, antidepressants, anticonvulsants, narcotic analgesics and narcotic antagonists, nonsteroidal antiinflammatory drugs (e.g., celecoxib), anticholinesterases, sympathomimetics and parasympathomimetics, ganglionic stimulating and blocking agents, neuromuscular blocking agents, antimuscarinic agents, adrenergic blocking agents, autacoids and autacoid antagonists, digitalis and digitalis congeners, diuretics and saliuretics, antibiotics and antimicrobials, cholesterol lowering agents, HMG CoA inhibitors (e.g., statins such as simvastatin, lovastatin, pravastatin, atorvastatin and the like) antineoplastics, immunosuppressants and immunomodulators, hemoglobin and hemoglobin derivatives and polymers, hormones and hormone antagonists, fat-soluble vitamins, and combinations thereof

A cosmetic active agent refers to a chemical or biological molecule having restorative, cleansing, protective, moisturizing, toning, conditioning or soothing effects, on skin, hair, or nails. Such cosmetic active agents may advantageously be included in various beauty care products including for example, day creams, night creams, makeup-removing creams, foundation creams, antisun creams, fluid foundations, makeup-removing milks, protective or body care milks, after-sun milks, skincare lotions, gels, mousses, cleansing lotions, antisun lotions, artificial tanning lotions, bath compositions, deodorizing compositions, aftershave gels and lotions and hair-removing creams.

An anti oxidant refers to a chemical or biological molecule having anti oxidant effects. Anti oxidants include for example, butylated hydroxytoluene (BHT), butylated hydroxy anisol (BHA) and carnosic acid.

A preservative refers to a chemical or biological molecule having inhibitory effects against microorganisms, including bacteria, viruses, fungi and molds. Preservatives include for example, methyl paraben, ethyl paraben, propyl paraben and butyl paraben.

A colorant refers to a chemical or biological molecule having pigmenting effects, and which is insoluble or has a low solubility in water.

A food additive refers to a chemical or biological molecule which is added to a processed food product. Food additives include for example, vitamins, preservatives, anti oxidants, flavouring agents.

An agriculturally active compound is a compound applied during agricultural procedures to the soil, water, air plant parts or plant products (seeds, fruits). These are either small organic molecule or biological molecules applied as pesticides, herbicides, compounds to prevent or combat disease, phytohormones and compounds applied to consumable parts of the plants.

A reagent used in chemical or biochemical reactions is a water-insoluble organic or biological molecule used as a starting material, reagent or catalyst. An example of such a reagent is a water-insoluble peptide.

A fragrance refers to a chemical or biological molecule which produces an olfactory effect. Fragrances include perfume oils such as natural aroma mixtures, such as those accessible from plant sources, for example pine, citrus, jasmine, patchouli, rose, or ylang-ylang oil. Also suitable are muscatel, salvia oil, chamomile oil, clove oil, lemon balm oil, mint oil, peppermint oil, spearmint oil, cinnamon leaf oil, linden blossom oil, juniper berry oil, vetiver oil, olibanum oil, galbanum oil, and labdanum oil, as well as orange blossom oil, neroli oil, orange peel oil, and sandalwood oil. Other suitable fragrances include but are not limited to fruits such as almond, apple, cherry, grape, pear, pineapple, orange, strawberry, raspberry; musk, flower scents such as lavender-like, rose-like, iris-like, and carnation-like. Other pleasant scents include herbal scents such as rosemary, thyme, and sage; and woodland scents derived from pine, spruce and other forest smells. A list of suitable fragrances is provided in U.S. Pat. Nos. 4,534,891, 5,112,688 and 5,145,842.

According to the invention described herein, the volatile water immiscible organic solvent is one which is effective for dissolution of the water-insoluble organic compound. Further, the volatile water immiscible organic solvent is volatile at the concentration used, such that it can be removed from the oil-in-water microemulsion in the second step of the processes described herein. The volatile water immiscible organic solvent for a preparation intended for pharmaceutical use, is one which is acceptable for administration to humans in trace amounts, if the redispersible powder or the aqueous dispersion comprising the water insoluble organic compound is intended for administration to humans. Appropriate volatile water immiscible organic solvents include for example, n-butyl acetate, sec-butyl acetate, isobutyl acetate, propyl acetate, toluene, xylenes, R(+)-limonene, hexane, pentane, heptane and mixtures thereof.

Alternatively, dissolution of the water-insoluble organic compound can be achieved using the volatile water immiscible organic solvent in combination with a co-solvent which is either miscible or immiscible with water. The co-solvent should be acceptable for administration to humans in trace amounts, if the redispersible powder or the aqueous dispersion comprising the water insoluble organic compound is intended for administration to humans. Suitable co-solvents include for example, ethanol, 1-propanol, 2-propanol, n-pentanol, n-butanol, ethyl acetate, tetrahydrofuran, propylene glycol, formamide, glycerol, polyethylene glycol and mixtures thereof.

According to the invention described herein, the surfactant is a surface-active agent which increases the emulsifying, foaming, dispersing, spreading and wetting properties of a product. The surfactant should further be acceptable for administration to humans if the redispersible powder or the aqueous dispersion comprising the water insoluble organic compound is intended for administration to humans. Suitable surfactants include cationic surfactants, anionic surfactants, amphoteric surfactants, nonionic surfactants, and mixtures thereof. Cationic surfactants include for example, cetyl trimethyl ammonium bromide, cetyl trimethyl ammonium chloride, and mixtures thereof. Anionic surfactants include for example, alkyl benzene sulphonates, sodium dodecyl sulfate, sodium sulfosuccinate, sodium lauryl sulfate, an alkyl naphthalene sulfonate condensate sodium salt, sodium stearate, and mixtures thereof. Amphoteric surfactants include various lecithins, such as egg lecithin, soya bean lecithin, synthetic saturated lecithins such as dimyristoyl phosphatidyl choline, dipalmitoyl phosphatidyl choline and distearoyl phosphatidyl choline, and synthetic unsaturated lecithins such as dioleyl phosphatidyl choline and dilinoleyl phosphatidyl choline. Nonionic surfactants include for example, ethoxylated sorbitan esters, sorbitan esters, polyglycerol esters, sucrose esters, poloxamers, alkyl polyglucosides, polyalkyleneoxide modified heptamethyltrisiloxanes, allyloxypolyethylene glycol methylethers and mixtures thereof.

According to the invention described herein, the re-dispersion aid is an agent which promotes dispersion of the powder of nanoparticles of the water-insoluble organic compound within an aqueous phase. Suitable dispersion aids include for example, wetting agents, disintegrants, water soluble polymers, colloidal silica particles, sugars, mannitol and mixtures thereof.

According to the invention described herein, a polymer is optionally included in the oil-in-water microemulsion The polymer can be a water insoluble polymer, including for example, polylactic acid, cellulose acetate, methyl cellulose, hydroxylpropyl methyl cellulose, poly(lactic-co-glycolic acid), hydroxylpropyl cellulose phthalate, and mixtures thereof. The polymer can be a water soluble polymer, including for example, polyvinyl pyrrolidone (PVP), polyvinyl alcohol, carboxy methyl cellulose, hydroxy ethyl cellulose, polyethylene glycol, gum arabic and mixtures thereof. The polymer is preferably a non-cross linked polymer. The polymer should further be acceptable for administration to humans if the redispersible powder or the aqueous dispersion comprising the water insoluble organic compound is intended for administration to humans.

To prepare the oil-in-water microemulsions as used in the invention, an organic phase and an aqueous phase are separately prepared and then mixed together to form the microemulsion. To prepare the organic phase, a water-insoluble organic compound is dissolved in a volatile water immiscible organic solvent, optionally in combination with a co-solvent. The aqueous phase is prepared by combination of the aqueous components, usually including the surfactant and water, and optionally in combination with a polymer and/or dispersion aid. Alternatively, the polymer, the re-dispersion aid and/or the surfactant can be mixed in the organic phase. The aforementioned dissolution steps can be spontaneous or can be carried out using various mechanical stirring instruments. The temperature and length of time for carrying out the dissolution steps can be adjusted as required to achieve improved results. The respective organic and aqueous phases so obtained are then mixed together to obtain a microemulsion. The microemulsion is formed spontaneously upon mixing of the phases by simple mechanical means such as vortexing. The temperature and length of time for carrying out the mixing of the phases can be adjusted as required to achieve improved results.

With regard to the methods for preparing a redispersible powder and for preparing an aqueous dispersion, the oil-in-water microemulsion can be prepared by a method which involves (i) dissolving the water-insoluble organic compound in the volatile water-immiscible organic solvent so as to form an organic phase; and (ii) mixing the organic phase with water and a surfactant so as to spontaneously form the oil-in-water microemulsion. The preparation of the microemulsion does not involve use of a high pressure homogenizer or a high shear instrument.

The size of the microemulsion droplets will be determined by the composition of the microemulsion and the temperature, since it is formed spontaneously.

The percent weight proportions of the various components used in the preparation of the microemulsion can be varied as required to achieve optimal results. The water-insoluble organic compound may be present in an amount of about 0.1 to about 20% by weight, the water-immiscible organic solvent may be present in an amount of about 0.5 to about 50% by weight, the surfactant may be present in an amount of about 5 to about 35% by weight and the water may be present in an amount of from about 20 to about 85% by weight, based on the total weight of the microemulsion. When a polymer is used in the preparation of the microemulsion, it may be present in an amount of about 0.01 to about 10% by weight based on the total weight of the microemulsion. When a co-solvent is used in the preparation of the microemulsion, it may be present in an amount of about 5 to about 30% by weight based on the total weight of the microemulsion. Alternate percent weight proportions are also envisioned. For example, the water-insoluble organic compound can be present in an amount of up to about 30% by weight; the water-immiscible organic solvent can be present in an amount of up to about 70% by weight; the surfactant can be present in an amount of up to about 40% by weight and the water can be present in an amount of from about 10 to about 90% by weight, based on the total weight of the microemulsion. In the method of preparing a redispersible powder, the final step involves removing the volatile water immiscible organic solvent and the water, thus yielding the redispersible powder. The solvent evaporation step can be sufficient to remove all of the volatile liquid components from the microemulsion, so that the powder has optimal handling characteristics e.g. is not "sticky", does not contain any potential breeding ground for contamination by microorganisms, and does not contain excessive amount of residual solvents. The removing step can be carried out by means known in the art, for example, lyophilization or spray drying.

For spray drying, the microemulsion can be directly filled into a laboratory spray dryer. Operating conditions can be varied according to the instrument and the experience of one skilled in the art. One set of operating conditions can include for example, air inlet temperature of 115-130 °C, air outlet temperature of 65-75 °C, feed rate of 28-30 mL/min and air flow rate of 450-550 m$^3$/h. The spray drying conditions should be set in such a way that the required properties of the resulting powder are met. For example, one may obtain a sticky solid if the air inlet temperature is too high compared to the melting points of the components, and by decreasing the inlet air temperature a free flowing powder can be obtained.

Optimal conditions, including for example time and temperature, for removing the organic volatile water-immiscible solvent and water can be determined empirically. The amount of organic solvent that remains after evaporation can be determined by HPLC.

The powder thus obtained upon removal of organic volatile water-immiscible solvent and water can be used as the final product, such as a powder of poorly soluble drug. It can also be re-dispersed in an aqueous medium, such as water, to yield an aqueous dispersion. The aqueous dispersion is usually obtained by gentle mixing, and is transparent and stable, such that there is no settling or precipitation.

In the method of preparing an aqueous dispersion, the final step involves removing the volatile water immiscible organic solvent, thus yielding the aqueous dispersion. The solvent evaporation step may be sufficient to remove the water immiscible organic solvent from the microemulsion, so that the aqueous dispersion does not contain excessive amount of residual solvents. In the disclosed method of preparing an aqueous dispersion, the solvent can be removed under reduced pressure, such as, by rotovap equipment.

After solvent evaporation and redispersal of the redispersible powder, the presence of nanoparticles can be ascertained microscopically by using cryo-transmission electron microscopy (Cryo-TEM). Prior to microscopic observation, a sample of the redispersable powder redispersed in water or the aqueous dispersion can be purified to remove excess surfactant. For purification, the sample can be ultrafiltered through a polysulfone membrane (cut off 300,000), washed with de-ionized water and centrifuged.

Afterwards, the sample is prepared for the observation in a Controlled Environment Vitrification Chamber. A thin film of the sample is prepared and immersed into liquid ethane at -183 °C. This procedure enables vitrification of the sample without any structural changes. The sample is kept at the temperature under -170° C for approximately 30 minutes till the equilibrium is reached, and then it is observed, while kept at low temperature.

The diameter size of the nanoparticles present in the redispersable powder or in the aqueous dispersion can be ascertained by light scattering measurements, for example using a dynamic light scattering instrument, such as the Zetasizer Nano ZS (Malvern Instruments, UK). The particle size can be determined either by volume distribution or by number distribution. The methods described herein generally yield nanoparticles of diameter less than 500 nm. The nanoparticles can be of diameter in the range 400 to 500 nm, 300 to 400 nm, 200 to 300 nm, 100 to 200 nm, 50 to 100 nm, 10 to 50 nm or 1 to 5 nm. In the methods and of the redispersible powder disclosed herein, the nanoparticles have a diameter of less than about 30 nm. The microemulsion droplets are typically below 30 nm in diameter.

The nanoparticles present in the redispersable powder or in the aqueous dispersion are in a particulate form. This means that the nanoparticles are discrete, individual, non-aggregated particle entities composed of a water-insoluble organic compound, such that the water-insoluble organic compound is not enclosed within, incorporated within, embedded within, contained within or associated with any encapsulation form, bead, carrier, matrix or similar delivery agent.

The nanoparticles comprise at least about 50% by weight of the water-insoluble organic compound. The nanoparticles may comprise at least about 80% by weight of the water-insoluble organic compound. The nanoparticles may consist essentially of the water-insoluble organic compound. The nanoparticles may comprise at least about 5% of the redispersible powder. The nanoparticles may comprise at least about 0.5% of the aqueous dispersion, or at least about 5.0% of the aqueous dispersion.

The active molecule may be present at 0.05%-90% of the total weight of the powder.

The methods of the invention described herein provide nanoparticles of a water-insoluble organic compound, which have significantly increased solubility and dissolution rate as compared to the same compound in unprocessed form, i.e., in a form which has not undergone any particle size reduction or other treatment to increase its solubility or dissolution rate. This is highly advantageous for the preparation of diverse consumer products, in which the active agents or other important components are usually insoluble or at best, poorly soluble. Thus, manufacturing and delivery solutions can be provided for example, for pharmaceutical compositions comprising poorly soluble drugs; for agricultural compositions e.g., poorly soluble pesticides, processed foods comprising poorly soluble preservatives, and cosmetics comprising poorly soluble active ingredients. The invention provides a means of providing such compounds at relatively high concentrations, compared to unprocessed forms.

Thus, the solubility of the water-insoluble organic compound can be at least about 5 times greater than the solubility of the water-insoluble organic compound in unprocessed form-i.e. not in the form of the nanoparticles prepared by the invention. The solubility of the water-insoluble organic compound can be at least about 10 times greater than the solubility of the water-insoluble organic compound in unprocessed form. The dissolution rate of the nanoparticles can be at least about 5 times greater than the dissolution rate of the water-insoluble organic compound in unprocessed form, or at least about 10 times greater than the dissolution rate of the water-insoluble organic compound in unprocessed form.

Thus, for example, if one milligram of a water-insoluble organic compound requires five minutes to dissolve, only one minute is required for the nanoparticles, at the same concentration. In addition, the solubility (gram material to gram water) also increases upon decreasing the size of the particles

In specific embodiments, the water-insoluble organic compound is in an amorphous or a partially amorphous form. Amorphous forms may have increased solubility relative to non-amorphous forms. Using amorphous forms of poorly soluble molecules can be a real advantage. Amorphous materials usually show a significantly higher solubility than their crystalline counterparts, have higher dissolution rate and, in case of drug entities, higher bioavailability in vivo. X-ray diffraction measurements and differential scanning calorimetry (DSC) measurements can be performed on the nanoparticles to reveal the presence of amorphous or crystalline materials.

In one embodiment of the method for preparing a redispersible powder, the process further comprises the step of crystallizing the nanoparticles thereby providing crystalline nanoparticles. In another embodiment, the crystallizing is carried out by aging the nanpoarticles. X-ray diffraction measurements can be performed on the nanoparticles to reveal crystallinity.

Also disclosed is a pharmaceutical composition comprising the redispersible powder of the invention and a suitable carrier. The carrier can be an aqueous medium, or a solid dosage form such as a tablet.

Pharmaceutical compositions can be manufactured by processes well known in the art, for example by means of conventional mixing, dissolving, granulating, grinding, pulverizing, dragee-making, levigating, emulsifying, encapsulating, entrapping or lyophilizing processes.

Pharmaceutical compositions comprising the redispersible powder of the invention can be formulated in conventional manner using one or more physiologically acceptable carriers comprising excipients and auxiliaries, which facilitate processing of the redispersible powder into preparations which, can be used pharmaceutically. Proper formulation is dependent upon the route of administration chosen.

For injection, the redispersible powder of the invention can be formulated as dispersions in aqueous media, preferably in physiologically compatible buffers such as Hank's solution, Ringer's solution, or physiological saline buffer. For transmucosal administration, penetrants appropriate to the barrier to be permeated are used in the formulation. Such penetrants, for example polyethylene glycol, are generally known in the art.

Solid pharmaceutical compositions which can be used orally, include tablets, capsules, pills, granules, powders, and the like. Tablets or granules can be produced by traditional wet granulation of the active substance in a kneader/mixer or by fluidized bed granulation. This entails use of the aqueous dispersion disclosed herein. Alternatively, a pharmaceutical substance can be mixed with the redispersible powder of the invention, and this mixture can be granulated with a solvent, preferably water or alcohols.

For administration by inhalation, the redispersible powders for use according to the present invention are conveniently delivered in the form of an aerosol spray presentation from a pressurized pack or a nebulizer with the use of a suitable propellant, e.g., dichlorodifluoromethane, trichlorofluoromethane, dichloro-tetrafluoroethane or carbon dioxide. In the case of a pressurized aerosol, the dosage unit can be determined by providing a valve to deliver a metered amount. Capsules and cartridges of, e.g., gelatin for use in an inhaler or insufflator, can be formulated containing a powder mix of the redispersible powder and a suitable powder base such as lactose or starch.

Pharmaceutical compositions for parenteral administration include aqueous dispersions of the redispersible powder. Additionally, suspensions of the redispersible powders can be prepared as appropriate aqueous injection suspensions. Suitable natural or synthetic carriers are well known in the art. Optionally, the suspension may also contain suitable stabilizers or agents, which increase the solubility of the compounds, to allow for the preparation of highly concentrated solutions.

A pharmaceutical composition may also be incorporated into an adhesive patch for transdermal drug delivery, as disclosed for example in Tan and Pfister (1999) Pharm Sci and Tech Today 2: 60-69.

As used herein, the term "excipient" refers to an inert substance added to the redispersible powder of the invention to further facilitate administration of the redispersible powder. Examples, without limitation, of excipients include calcium carbonate, calcium phosphate, various sugars and types of starch, cellulose derivatives, gelatin, vegetable oils and polyethylene glycols. Pharmaceutical compositions may also include one or more additional active ingredients.

Pharmaceutical compositions suitable for use in context of the present invention include compositions wherein the active ingredients comprising the water-insoluble organic compound are contained in an amount effective to achieve the intended purpose. All formulations for administration should be in dosages suitable for the chosen route of administration. More specifically, a "therapeutically effective" dose means an amount of a compound effective to prevent, alleviate or ameliorate symptoms of a disease of the subject being treated. Determination of a therapeutically effective amount is well within the capability of those skilled in the art, especially in light of the detailed disclosure provided herein.

The foregoing description of the specific embodiments will so fully reveal the general nature of the invention that others can, by applying current knowledge, readily modify and/or adapt for various applications such specific embodiments without undue experimentation and without departing from the brand concept, and, therefore, such adaptations and modifications should and are intended to be comprehended within the meaning and range of equivalents of the disclosed embodiments. It is to be understood that the phraseology or terminology employed herein is for the purpose of description and not of limitation. The means, materials, and steps for carrying out various disclosed functions may take a variety of alternative forms without departing from the invention.

The following examples are presented in order to more fully illustrate certain embodiments of the invention. They should in no way, however, be construed as limiting the broad scope of the invention.

## EXAMPLES

### EXAMPLE 1

An oil-in-water microemulsion was prepared having the indicated percent weight proportions of the following materials: sodium dodecyl sulfate (8%), n-butyl acetate (3.5%), 2-propanol (3.5%), water (82%) and propyl paraben (3%).

The microemulsion was prepared by first dissolving the required quantity of propyl paraben in the mixture of n-butyl acetate and 2-propanol to prepare an organic phase. Next, sodium dodecyl sulfate (surfactant) was dissolved in water to prepare an aqueous phase. Aqueous and organic phases were mixed together and vortexed until a transparent microemulsion was formed.

### EXAMPLE 2

An oil-in-water microemulsion was prepared having the indicated percent weight proportions of the following materials: sodium dodecyl sulfate (9%), n-butyl acetate (10%), 2-propanol (10%), water (61%), polyvinylpyrrolidone 40000 (PVP)(7%) and propyl paraben (3%).

The microemulsion was prepared by first dissolving the required quantity of propyl paraben in the mixture of n-butyl acetate and 2-propanol to prepare an organic phase. Next, sodium dodecyl sulfate (surfactant) and PVP were dissolved in water and mixed for 10 min to prepare an aqueous phase. Aqueous and organic phases were mixed together and vortexed until a transparent microemulsion was formed.

After removing the volatile solvents under reduced pressure, nanoparticles of 7 nm were formed.

### EXAMPLE 3

An oil-in-water microemulsion was prepared having the indicated percent weight proportions of the following materials: sodium dodecyl sulfate (8%), n-butyl acetate (3.5%), 2-propanol (3.5%), water (82%) and propyl paraben (3%).

The microemulsion was prepared by first dissolving the required quantity of propyl paraben in the mixture of n-butyl acetate and 2-propanol to prepare an organic phase. Next, sodium dodecyl sulfate (surfactant) was dissolved in water to prepare an aqueous phase. Aqueous and organic phases were mixed together and vortexed until a transparent microemulsion was formed.

The microemulsion was then spray dried into a laboratory spray drier and the resulting redispersible powder could be stored for month in a sealed vial. The powder was composed of 27% propyl paraben and 73% sodium dodecyl sulfate according to weight. The powder was easily redispersible in water up to 5% according to weight by adding 3% PVP, and formed a transparent dispersion of nanoparticles sized 8-9 nm.

EXAMPLE 4

An oil-in-water microemulsion was prepared having the indicated percent weight proportions of the following materials: sodium dodecyl sulfate (9%), n-butyl acetate (4.5%), 2-propanol (4.5%), water (66.9%), PVP (9.1%) and butyl paraben (6%).
The microemulsion was prepared by first dissolving the required quantity of butyl paraben in the mixture of n-butyl acetate and 2-propanol to prepare an organic phase. Next, sodium dodecyl sulfate (surfactant) and PVP were dissolved in water and mixed for 10 min to prepare an aqueous phase. Aqueous and organic phases were mixed together and vortexed until a transparent microemulsion was formed.
The microemulsion was then spray dried into a laboratory spray drier and the resulting powder could be stored for month in a sealed vial. The powder was composed of 37.3% sodium dodecyl sulfate, 24.9% butyl paraben and 37.8% PVP according to weight. The powder was easily redispersible in water up to 5% according to weight and formed a transparent dispersion of nanoparticles.

EXAMPLE 5

An oil-in-water microemulsion was prepared having the indicated percent weight proportions of the following materials: sodium dodecyl sulfate (18%), n-butyl acetate (4.5%), ethanol (4.5%), water (72%), carnosic acid (1%)
The microemulsion was prepared by first dissolving carnosic acid in the mixture of n-butyl acetate and ethanol to prepare an organic phase. Next, sodium dodecyl sulfate (surfactant) was dissolved in water and mixed for 4 min to prepare an aqueous phase. Aqueous and organic phases were mixed together and vortexed until a clear microemulsion was formed.
The microemulsion was lyophilized and the resulting powder could be stored for months in a sealed vial. The powder was composed of 5.3% carnosic acid and 94.7% sodium dodecyl sulfate according to weight. The powder was easily redispersible in water up to 20% according to weight and formed a dispersion of nanoparticles of 70 nm in diameter. A similar process can be performed by a means of spray drier.

EXAMPLE 6

An oil-in-water microemulsion was prepared having the indicated percent weight proportions of the following materials: soybean lecithin (Emultop® HL 50; 14.4%), Tween® 80 (20%), n-butyl acetate (6%), 2-propanol (12%), mannitol (5%) water (38.4%) and propyl paraben (4.2%).
The microemulsion was prepared by first dissolving the required quantity of propyl paraben in the mixture of n-butyl acetate and 2-propanol to prepare an organic phase. Next, the organic phase was added to a mixture of soybean lecithin and Tween® 80 and gently heated to 40° for 10 minutes. Mannitol was dissolved in water to form an aqueous phase, and the two phases were mixed together and vortexed until a clear microemulsion was formed.
The microemulsion was spray dried in a laboratory spray dryer to yield an easily dispersible "cake". It was composed of 9.6% propyl paraben according to weight, was easily redispersible up to 5% in water, and formed a stable dispersion of nanoparticles of 100 nm in diameter.

EXAMPLE 7

An oil-in-water microemulsion was prepared having the indicated per cent weight proportions of the following materials: soybean lecithin (Emultop® HL 50; 14.4%), Tween® 80 (2%), toluene (10%), 2-propanol (14%), mannitol (5%), water (36.6%) and beta-carotene (233 ppm).
The microemulsion was prepared by first dissolving beta-carotene in toluene to prepare an organic phase. Then, this solution was added to a mixture of Tween® 80, soybean lecithin and 2-propanol. Mannitol was dissolved in water to form an aqueous phase, and two phases were mixed together and vortexed with gentle heating to 40° for 10 minutes until a clear, stable microemulsion was formed. The microemulsion showed high electric conductivity, implying oil-in-water character.
The microemulsion was spray dried in a laboratory spray dryer to yield an easily dispersible "oily cake". It contained 591 ppm of beta-carotene and was easily redispersible in water up to 5% according to weight, and formed a stable dispersion of nanoparticles of 100 nm and a small fraction of 230 nm particles in diameter.

EXAMPLE 8

An oil-in-water microemulsion was prepared having the indicated percent weight proportions of the following materials: Tween® 80 (22.5%), sec-butyl acetate (7%), Ethanol (22.5%), water (45%) and butylated hydroxy toluene (BHT) (3%).

The microemulsion was prepared by first dissolving the required quantity of BHT in sec-butyl acetate to prepare an organic phase. Next, Tween® 80 was dissolved in water and ethanol was added to prepare an aqueous phase. Aqueous and organic phases were mixed together and vortexed until a transparent microemulsion was formed.

After removing the volatile solvents under reduced pressure, nanoparticles of 12nm were formed, dispersed in water.

EXAMPLE 9

An oil-in-water microemulsion was prepared having the indicated percent weight proportions of the following materials: Pluronic® F68 (22.5%), sec-butyl acetate (5%), ethanol (22.5%), water (45%) and butylated hydroxy anisol (BHA) (5%).

The microemulsion was prepared by first dissolving the required quantity of BHA in sec-butyl acetate to prepare an organic phase. Next, Pluronic® F68 was dissolved in water and ethanol was added to prepare an aqueous phase. Aqueous and organic phases were mixed together and vortexed until a transparent microemulsion was formed.

The microemulsion was then lyophilized. The resulting powder was composed of nanoparticles sized 16-25 nm, containing 18.2% BHA and 81.8% Pluronic® F68.

EXAMPLE 10

An oil-in-water microemulsion was prepared having the indicated percent weight proportions of the following materials: Tween® 80 (7%), soybean lecithin (P5638 (Sigma; 3%), sodium dodecyl sulfate (2.3%), n-butyl acetate (16%), ethanol (16%), polyvinylpyrrolidone 40000 (PVP)(5%), mannitol (2%), water (45.7%) and celecoxib (3%).

The microemulsion was prepared by first dissolving the required quantity of celecoxib in the mixture of n-butyl acetate and ethanol. Tween® 80 and soybean lecithin (surfactants) were dispersed in the resulting solution to prepare an organic phase. Next, sodium dodecyl sulfate (surfactant), mannitol and PVP were dissolved in water and mixed for 10 min to prepare an aqueous phase. Aqueous and organic phases were mixed together and vortexed until a transparent microemulsion was formed.

The microemulsion was then spray dried by a laboratory spray drier. The powder was composed of 13.45% celecoxib, 13.45% soybean lecithin, 31.4% Tween® 80, 10.3% sodium dodecyl sulfate, 22.4% polyvinylpyrrolidone 40000 (PVP) and 9% mannitol according to weight. The powder was easily redispersible in water and formed a stable dispersion of nanoparticles of 57 nm in diameter, as determined by number distribution in light scattering measurements.

EXAMPLE 11

An oil-in-water microemulsion was prepared having the indicated percent weight proportions of the following materials: Tween® 80 (10%), sodium cholate (4%), polyvinylpyrrolidone 10000 (PVP) (4%), sec-butyl acetate (8%), isopropyl alcohol (22.5%), water (46%) and carnosic acid (5.5%).

The microemulsion was prepared by first dissolving the required quantity of carnosic acid in isopropyl alcohol. Next, sec-butyl acetate was added to the resulting solution to prepare an oily phase. Tween® 80, sodium cholate (surfactants), and PVP were dissolved in water to prepare an aqueous phase. Aqueous and organic phases were mixed together and vortexed until a transparent microemulsion was formed.

The microemulsion was then spray dried by a laboratory spray drier. The powder was composed of 23% carnosic acid, 17% sodium cholate, 43% Tween® 80 and 17% PVP according to weight. The powder was easily redispersible in water. and formed a stable dispersion of nanoparticles having an average diameter of 63 nm, as determined by number distribution in light scattering measurements.

EXAMPLE 12

An oil-in-water microemulsion was prepared having the indicated percent weight proportions of the following materials: Tween® 80 (14%), soybean lecithin (Emultop® HL 50; 14%), n-butyl acetate (8%), ethanol (19%), hydrochloric acid (0.06%), sucrose (10%), water (29.9%) and simvastatin (5%).

The microemulsion was prepared by first dissolving the required quantity of simvastatin in the mixture of n-butyl acetate and ethanol. Afterwards, Tween® 80 and soybean lecithin (surfactants) were dispersed in the resulting solution to prepare an organic phase. Next, sucrose and hydrochloric acid were dissolved in water to prepare an aqueous phase. Aqueous and organic phases were mixed together and vortexed until a transparent microemulsion was formed.

The microemulsion was then lyophilized and the resulting redispersible powder contained 11% simvastatin according to weight. The powder was easily redispersible in water up to 5% according to weight. The majority of the resulting nanoparticles had an average size of 62 nm, as determined by number distribution in light scattering measurements.

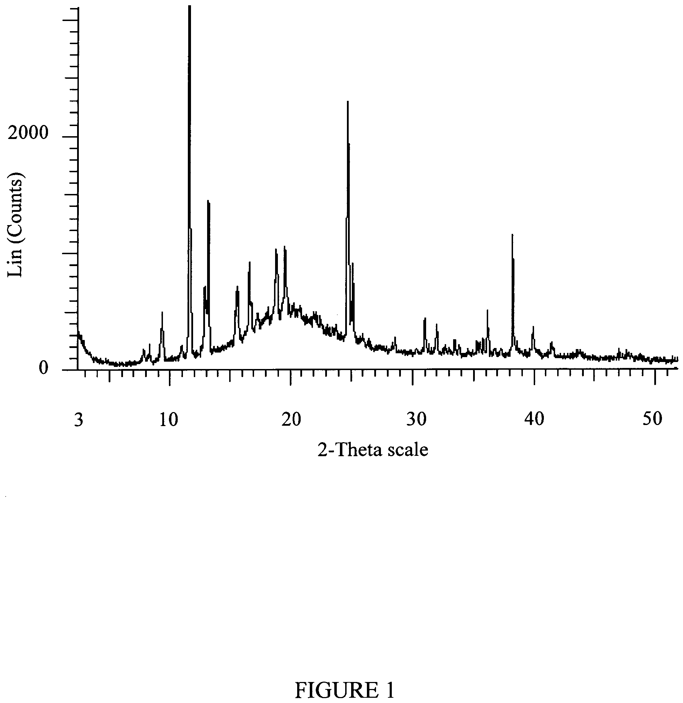

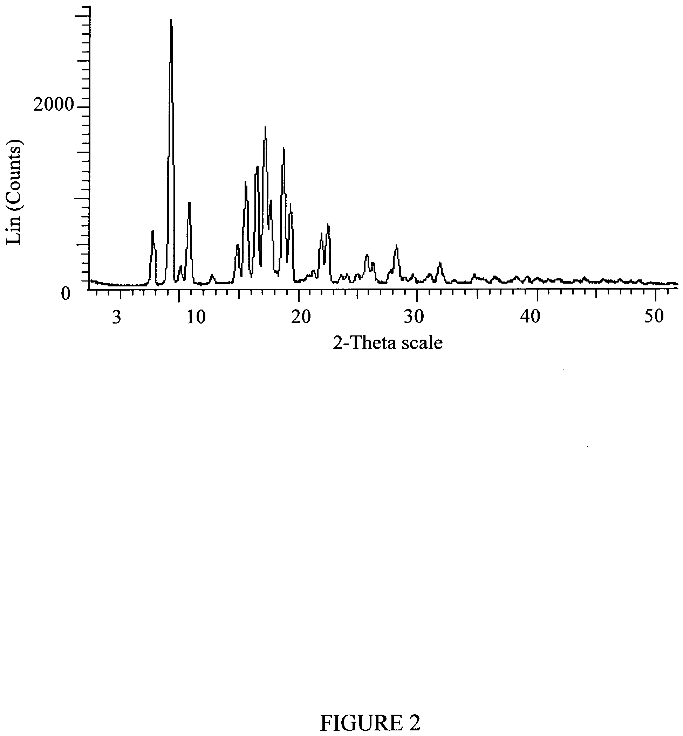

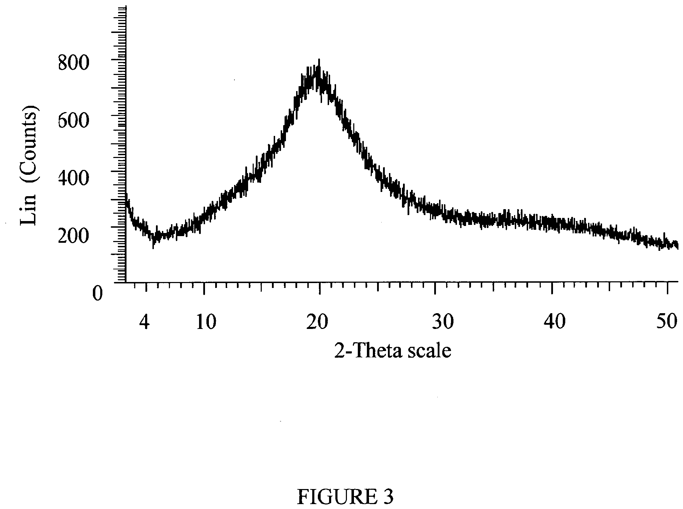

In order to evaluate the crystallinity of the simvastatin nanoparticles, X-ray diffraction measurements were performed on simvastatin samples as follows: (a) raw material crystalline simvastatin in the absence of any additional ingredients or treatment; (b) crystalline simvastatin in combination with the additional ingredients of the formulation, but prior to formulation (physical mix), and (c) the simvastatin nanoparticle formulation, prepared as in this Example. The measurements provide definitive evidence that the active water-insoluble organic compound (simvastatin) is in an amorphous form following formulation. The peaks of crystalline simvastatin appear in the physical mix diffractogram (Fig. 1) and in the simvastatin alone diffractogram (Fig. 2). Physical mix refers to the mixture of all components comprising the simvastatin nanoparticle formulation, in the same proportions by weight, in their unprocessed form. In contrast, there are no peaks of crystalline simvastatin in the nanoparticles diffractogram (Fig.3). This proves the totally amorphous character of the water-insoluble organic compound simvastatin in the nanoparticle formulation.

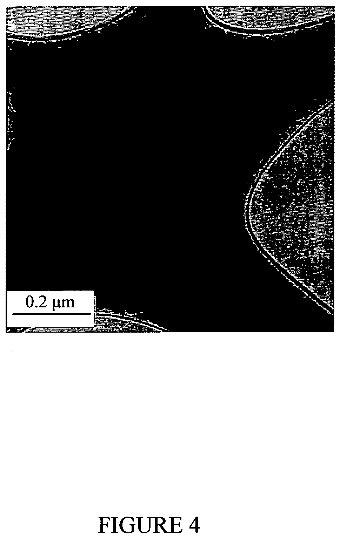

In order to ascertain the formation of nanometric particles, the resulting simvastatin powder was dispersed in water and examined using cryo-transmission electron microscopy (Cryo-TEM). The Cryo-TEM image in Fig. 4 indicates that the particles have a size in the nanometric range.

In order to evaluate presence of simvastatin within the nanoparticles, the following experiment was conducted. After lyophilization of the microemulsion described in this Example, the resulting powder formed therefrom was dispersed in water (0.5% wt), vortexed for 2 minutes, stirred for 20 minutes and filtered through 0.45 micron filter. The concentration of the simvastatin in the filtrate was evaluated using HPLC equipped with a 238nm detector and RP 18 column. It was found that 98% of the simvastatin which was present in the powder, was found in the filtrate. A control experiment of simvastatin solubilized in a mixture of surfactants dispersed in water at the same concentrations was conducted. The active material and the surfactants were stirred together in water for 48 hours. Afterwards, the dispersion was filtered through a 0.45 micron filter and the concentration of the simvastatin in the filtrate was evaluated using the same system. Only 24% of simvastatin was present in the filtrate in the control experiment and was probably solubilized in the mixture of the surfactants.

**Claims**

1.    A process for preparing a redispersible powder comprising nanoparticles of a water-insoluble organic compound, the nanoparticles comprising at least 50% by weight of the water-insoluble organic compound, the process comprising the steps of:

(i) preparing an oil-in-water microemulsion comprising a water-insoluble organic compound, a volatile water-immiscible organic solvent, water, and at least one surfactant, by:

(a) dissolving the water-insoluble organic compound in the volatile water-immiscible organic solvent so as to form an organic phase; and
(b) mixing the organic phase with water and at least one surfactant so as to spontaneously form the oil-in-water microemulsion without the use of a high pressure homogenizer or a high shear instrument;

wherein the diameter of the microemulsion droplets is below 30 nm; and
(ii) removing the volatile water-immiscible organic solvent and the water so as to form the redispersible powder comprising said nanoparticles, wherein the volatile water-immiscible organic solvent and the water are removed simultaneously, and

wherein said nanoparticles are in a particulate form and have a diameter of less than 30 nanometers.

2.    The process according to claim 1, wherein the volatile water-immiscible organic solvent and the water are removed by spray drying or lyophilization

3.    The process according to claim 1, wherein the water-insoluble organic compound is selected from the group consisting of pharmaceutically active agents, cosmetic active agents, anti-oxidants, preservatives, colorants, food additives, agriculturally active compounds, reagents used in chemical and biochemical reactions and fragrances, preferably wherein the pharmaceutically active agent is a statin.

4.    The process according to claim 1,
wherein the microemulsion further comprises a re-dispersion aid; preferably wherein the re-dispersion aid is selected from the group consisting of a wetting agent, a disintegrant, a water soluble polymer, colloidal silica particles, sugars, mannitol, lactose and mixtures thereof; or
wherein the microemulsion further comprises a co-solvent.

5. The process according to claim 1, further comprising the step of re-dispersing the powder in water to form an aqueous dispersion of the nanoparticles.

6. The process according to claim 1, wherein the microemulsion further comprises at least one polymer.

7. The process according to claim 6,
wherein the polymer is a water insoluble polymer selected from the group consisting of polylactic acid, cellulose acetate, methyl cellulose, hydroxylpropyl methyl cellulose, poly(lactic-co-glycolic acid), hydroxylpropyl cellulose phthalate, and mixtures thereof; or
wherein the polymer is a water soluble polymer selected from the group consisting of polyvinyl pyrrolidone (PVP), polyvinyl alcohol, carboxy methyl cellulose, hydroxy ethyl cellulose, polyethylene glycol, gum arabic and mixtures thereof; or
wherein the polymer is a non-crosslinked polymer; or
wherein the polymer is present in an amount of 0.01 to 10% by weight based on the total weight of the microemulsion.

8. The process according to claim 1, wherein the surfactant is selected from the group consisting of a cationic surfactant, an anionic surfactant, an amphoteric surfactant, a nonionic surfactant and mixtures thereof.

9. The process according to claim 8, wherein
the anionic surfactant is selected from the group consisting of an alkyl benzene sulphonate, sodium dodecyl sulfate, sodium sulfosuccinate, sodium lauryl sulfate, alkyl naphthalene sulfonate condensate sodium salt, sodium stearate, and mixtures thereof;
the nonionic surfactant is selected from the group consisting of an ethoxylated sorbitan ester, a sorbitan ester, a polyglycerol ester, a sucrose ester, a poloxamer, an alkyl polyglucoside, a polyalkyleneoxide modified heptamethyltrisiloxane, an allyloxypolyethylene glycol methylether and mixtures thereof;

the amphoteric surfactant is lecithin; and
the cationic surfactant is selected from the group consisting of cetyl trimethyl ammonium bromide, cetyl trimethyl ammonium chloride, and mixtures thereof.

10. The process according to claim 1, wherein the water-insoluble organic compound is in an amorphous or a partially amorphous form.

11. The process according to claim 1, further comprising the step of crystallizing the nanoparticles, to provide crystalline organic nanoparticles, preferably wherein the nanoparticles are crystallized by aging.

12. A redispersible powder comprising nanoparticles of a water-insoluble organic compound, the nanoparticles having a diameter of less than 30 nanometers and comprising at least 50% by weight of the water-insoluble organic compound, wherein the redispersible powder is prepared by a process comprising the steps of:

(i) preparing an oil-in-water microemulsion comprising a water-insoluble organic compound, a volatile water-immiscible organic solvent, water, and at least one surfactant, by:

(a) dissolving the water-insoluble organic compound in the volatile water-immiscible organic solvent so as to form an organic phase; and
(b) mixing the organic phase with water and at least one surfactant so as to spontaneously form the oil-in-water microemulsion without the use of a high pressure homogenizer or a high shear instrument;

wherein the diameter of the microemulsion droplets is below 30nm; and
(ii) removing the volatile water-immiscible organic solvent and the water so as to form the redispersible powder comprising said nanoparticles, wherein said nanoparticles are in a particulate form, wherein the volatile water-immiscible organic solvent and the water are removed simultaneously.

13. The redispersible powder according to claim 12, wherein the powder is capable of being re-dispersed in water to form an aqueous dispersion of the nanoparticles.

14. The redispersible powder according to claim 12, comprising at least 10% by weight of nanoparticles of a water-insoluble organic compound, wherein the nanoparticles are in a particulate form.

15. The redispersible powder according to claim 12,
wherein the water-insoluble organic compound is selected from the group consisting of pharmaceutically active agents, cosmetic active agents, anti-oxidants, preservatives, colorants, food additives, agriculturally active compounds, reagents used in chemical and biochemical reactions and fragrances; or
wherein the water-insoluble organic compound is in an amorphous form or a partially amorphous form; or
further comprising at least one polymer.


**Patentansprüche**

1. Verfahren zur Herstellung eines redispergierbaren Pulvers, das Nanoteilchen einer Wasser-unlöslichen organischen Verbindung umfasst, worin die Nanoteilchen mindestens 50 Gew.-% der Wasser-unlöslichen organischen Verbindung enthalten, wobei das Verfahren die Schritte umfasst:

   (i) Herstellen einer Öl-in-Wasser Mikroemulsion, welche enthält, eine Wasser-unlösliche organische Verbindung, ein flüchtiges mit Wasser nicht-mischbares organisches Lösungsmittel, Wasser, und mindestens ein oberflächenaktives Mittel, durch:

      (a) Lösen der Wasser-unlöslichen organischen Verbindung in dem flüchtigen, mit Wasser nicht-mischbaren organischen Lösungsmittel, um eine organische Phase zu bilden; und
      (b) Mischen der organischen Phase mit Wasser und mindestens einem oberflächenaktiven Mittel, um die Öl-in-Wasser Mikroemulsion spontan ohne Verwendung eines Hochdruck-Homogenisators oder eines eine hohe Scherkraft liefernden Instruments zu bilden;

   wobei der Durchmesser der Mikroemulsions-Tröpfchen unter 30 nm liegt; und
   (ii) Entfernen des flüchtigen, mit Wasser nicht-mischbaren organischen Lösungsmittels und des Wassers, um das redispergierbare Pulver mit den Nanoteilchen zu bilden, wobei das flüchtige, mit Wasser nicht-mischbare organische Lösungsmittel und das Wasser gleichzeitig entfernt werden, und

   wobei die Nanoteilchen in Teilchenform vorliegen und einen Durchmesser von kleiner als 30 Nanometer aufweisen.

2. Verfahren nach Anspruch 1, wobei das flüchtige, mit Wasser nicht-mischbare organische Lösungsmittel und das Wasser durch Sprühtrocknen oder Lyophilisieren entfernt werden.

3. Verfahren nach Anspruch 1, wobei die Wasser-unlösliche organische Verbindung ausgewählt ist aus der Gruppe bestehend aus pharmazeutisch aktiven Mitteln, kosmetischen Mitteln, aktiven Mitteln, Antioxidantien, Konservierungsmitteln, Farbstoffen, Nahrungsmittelzusätzen, landwirtschaftlich aktiven Verbindungen, in chemischen und biochemischen Reaktionen und Duftsoffen verwendeten Reagenzien, vorzugsweise wobei das pharmazeutisch aktive Mittel ein Statin ist.

4. Verfahren nach Anspruch 1,
wobei die Mikroemulsion weiter eine Re-Dispersions-Hilfe umfasst; vorzugsweise wobei die Re-Dispersions-Hilfe ausgewählt ist aus der Gruppe bestehend aus einem Benetzungsmittel, einem Desintegrierungsmittel, einem Wasser-löslichen Polymer, kolloidalen Siliziumdioxid-Teilchen, Zuckern, Mannitol, Laktose und Gemischen davon; oder
wobei die Mikroemulsion weiter ein Co-Lösungsmittel umfasst.

5. Verfahren nach Anspruch 1, welches weiter den Schritt umfasst, erneutes Dispergieren des Pulvers in Wasser, um eine wässrige Dispersion von Nanopartikeln zu bilden.

6. Verfahren nach Anspruch 1, wobei die Mikroemulsion weiter mindestens ein Polymer umfasst.

7. Verfahren nach Anspruch 6,
wobei das Polymer ein in Wasser unlösliches Polymer ist ausgewählt aus der Gruppe bestehend aus Polymilchsäure, Celluloseacetat, Methylcellulose, Hydroxylpropylmethylcellulose, Poly(milchsäure-co-glycolsäure), Hydroxylpropylcellulosephthalat und Gemischen davon; oder

wobei das Polymer ein Wasser-lösliches Polymer ist, ausgewählt aus der Gruppe bestehend aus Polyvinylpyrrolidon (PVP), Polyvinylalkohol, Carboxymethylcellulose, Hydroxyethylcellulose, Polyethyleneglycol, Gum Arabicum and Gemischen davon; oder

wobei das Polymer ein nicht-vernetztes Polymer ist; oder

wobei das Polymer in einer Menge vorhanden ist von etwa 0,01 bis etwa 10 Gew.-% bezogen auf das Gesamtgewicht der Mikroemulsion.

8. Verfahren nach Anspruch 1, wobei das oberflächenaktive Mittel ausgewählt ist aus der Gruppe bestehend aus einem kationischen oberflächenaktiven Mittel, einem anionischen oberflächenaktiven Mittel, einem amphoteren oberflächenaktiven Mittel, einem nichtionischen oberflächenaktiven Mittel und Gemischen davon.

9. Verfahren nach Anspruch 8, wobei

das anionische oberflächenaktive Mittel ausgewählt ist aus der Gruppe bestehend aus einem Alkylbenzolsulphonat, Natriumdodecylsulfat, Natriumsulfosuccinat, Natriumlaurylsulfat, Alkylnaphthalensulfonat-Kondensat Natriumsalz, Natriumastearat und Gemischen davon;

das nichtionische oberflächenaktive Mittel ausgewählt ist aus der Gruppe bestehend aus einem ethoxylierten Sorbitester, einem Sorbitester, einem Polyglycerinester, einem Saccharoseester, einem Poloxamer, einem Alkylpolyglucosid, einem Polyalkyleneoxid-modifizierten Heptamethyltrisiloxan, einem Allyloxypolyethylenglycolmethylether und Gemischen davon;

das amphotere oberflächenaktive Mittel Lecithin ist; und

das kationische oberflächenaktive Mittel ausgewählt ist aus der Gruppe bestehend aus Cetyltrimethylammoniumbromid, Cetyltrimethylammoniumchlorid und Gemischen davon.

10. Verfahren nach Anspruch 1, wobei die Wasser-unlösliche organische Verbindung in einer amorphen oder einer teilweise amorphen Form vorliegt.

11. Verfahren nach Anspruch 1, welches weiter den Schritt umfasst, Kristallisieren der Nanoteilchen, um kristalline organische Nanoteilchen zu liefern, vorzugsweise wobei die Nanoteilchen durch Altern kristallisiert werden.

12. Redispergierbares Pulver, welches Nanoteilchen einer Wasser-unlöslichen organischen Verbindung umfasst, worin die Nanoteilchen einen Durchmesser von kleiner als etwa 30 Nanometer aufweisen und mindestens 50 Gew.-% der Wasser-unlöslichen organischen Verbindung enthalten, worin das redispergierbare Pulver hergestellt wird durch ein Verfahren, welches die Schritte umfasst:

(i) Herstellen einer Öl-in-Wasser Mikroemulsion, die enthält, eine Wasser-unlösliche organische Verbindung, ein flüchtiges, mit Wasser nicht-mischbares organisches Lösungsmittel, Wasser, und mindestens ein oberflächenaktives Mittel, durch:

(a) Lösen der Wasser-unlöslichen organischen Verbindung in dem flüchtigen, mit Wasser nicht-mischbaren organischen Lösungsmittel, um eine organische Phase zu bilden; und

(b) Mischen der organischen Phase mit Wasser und mindestens einem oberflächenaktiven Mittel, um die Öl-in-Wasser Mikroemulsion spontan ohne Verwendung eines Hochdruck-Homogenisators oder eines eine hohe Scherkraft liefernden Instruments zu bilden;

wobei der Durchmesser der Mikroemulsion-Tröpfchen unter 30nm liegt; und

(ii) Entfernen des flüchtigen, mit Wasser nicht-mischbaren organischen Lösungsmittels und des Wassers, um das redispergierbare Pulver mit den Nanoteilchen zu bilden, wobei die Nanoteilchen in einer Teilchen-Form sind, wobei das flüchtige, mit Wasser nicht-mischbare organische Lösungsmittel und das Wasser gleichzeitig entfernt werden.

13. Redispergierbares Pulver nach Anspruch 12, wobei das Pulver in Wasser redispergiert werden kann, um eine wässrige Dispersion der Nanoteilchen zu bilden.

14. Redispergierbares Pulver nach Anspruch 12, welches mindestens 10 Gew.-% Nanoteilchen einer Wasser-unlöslichen organischen Verbindung enthält, wobei die Nanoteilchen in einer Teilchenform vorliegen.

**15.** Redispergierbares Pulver nach Anspruch 12,
wobei die Wasser-unlösliche organische Verbindung ausgewählt ist aus der Gruppe bestehend aus pharmazeutisch aktiven Mitteln, kosmetischen Mitteln, aktiven Mitteln, Antioxidantien, Konservierungsmitteln, Farbstoffen, Nahrungsmittelzusätzen, landwirtschaftlich aktiven Verbindungen, in chemischen und biochemischen Reaktionen und Duftsoffen verwendeten Reagenzien;
wobei die Wasser-unlösliche organische Verbindung in einer amorphen Form oder einer teilweise amorphen Form vorliegt; oder
welches weiter mindestens ein Polymer umfasst.

**Revendications**

**1.** Procédé de préparation d'une poudre redispersable comprenant des nanoparticules d'un composé organique insoluble dans l'eau, les nanoparticules comprenant au moins 50 % en poids du composé organique insoluble dans l'eau, le procédé comprenant les étapes consistant à :

(i) préparer une microémulsion huile dans l'eau comprenant un composé organique insoluble dans l'eau, un solvant organique volatil non miscible à l'eau, de l'eau, et au moins un tensioactif, par :

(a) dissolution du composé organique insoluble dans l'eau dans le solvant organique volatil non miscible dans l'eau de manière à former une phase organique ; et
(b) mélange de la phase organique avec de l'eau et au moins un tensioactif de manière à former spontanément la microémulsion huile dans l'eau sans l'utilisation d'un homogénéisateur haute pression ou d'un instrument à cisaillement élevé ;

dans lequel le diamètre des gouttelettes de microémulsion est inférieur à 30 nm ; et
(ii) à éliminer le solvant organique volatil non miscible dans l'eau et l'eau de manière à former la poudre redispersable comprenant lesdites nanoparticules, le solvant organique volatil non miscible à l'eau et l'eau étant éliminés simultanément, et

dans lequel lesdites nanoparticules sont sous une forme particulaire et ont un diamètre inférieur à 30 nanomètres.

**2.** Procédé selon la revendication 1, dans lequel le solvant organique volatil non miscible à l'eau et l'eau sont éliminés par séchage par pulvérisation ou lyophilisation.

**3.** Procédé selon la revendication 1, dans lequel le composé organique insoluble dans l'eau est choisi dans le groupe constitué par des agents pharmaceutiquement actifs, des agents actifs cosmétiques, des antioxydants, des conservateurs, des colorants, des additifs alimentaires, des composés actifs dans le domaine agricole, des réactifs utilisés dans des réactions chimiques et biochimiques et des parfums, dans lequel, de préférence, l'agent pharmaceutiquement actif est une statine.

**4.** Procédé selon la revendication 1,
dans lequel la microémulsion comprend, en outre, un auxiliaire de redispersion ; dans lequel, de préférence, l'auxiliaire de redispersion est choisi dans le groupe constitué par un agent mouillant, un délitant, un polymère hydrosoluble, des particules de silice colloïdale, des sucres, du mannitol, du lactose et des mélanges de ceux-ci ; ou
dans lequel la microémulsion comprend, en outre, un co-solvant.

**5.** Procédé selon la revendication 1 comprenant, en outre, l'étape de redispersion de la poudre dans de l'eau pour former une dispersion aqueuse des nanoparticules.

**6.** Procédé selon la revendication 1, dans lequel la microémulsion comprend, en outre, au moins un polymère.

**7.** Procédé selon la revendication 6,
dans lequel le polymère est un polymère insoluble dans l'eau choisi dans le groupe constitué par l'acide polylactique, l'acétate de cellulose, la méthylcellulose, l'hydroxylpropylméthyl cellulose, l'acide poly (lactique-co-glycolique), le phtalate d'hydroxylpropyl cellulose et des mélanges de ceux-ci ; ou
dans lequel le polymère est un polymère hydrosoluble choisi dans le groupe constitué par la polyvinylpyrrolidone (PVP), l'alcool polyvinylique, la carboxyméthylcellulose, l'hydroxyéthylcellulose, le polyéthylène glycol, la gomme arabique et des mélanges de ceux-ci ; ou

dans lequel le polymère est un polymère non réticulé ; ou

dans lequel le polymère est présent en une quantité de 0,01 à 10 % en poids sur la base du poids total de la microémulsion.

8. Procédé selon la revendication 1, dans lequel le tensioactif est choisi dans le groupe constitué par un tensioactif cationique, un tensioactif anionique, un tensioactif amphotère, un tensioactif non ionique et des mélanges de ceux-ci.

9. Procédé selon la revendication 8, dans lequel
le tensioactif anionique est choisi dans le groupe constitué par un alkylbenzène sulfonate, le dodécylsulfate de sodium, le sulfosuccinate de sodium, le lauryl sulfate de sodium, l'alkyle, le sel de sodium de condensat de sulfonate de naphtalène, le stéarate de sodium et des mélanges de ceux-ci ;
le tensioactif non ionique est choisi dans le groupe constitué par un ester de sorbitan éthoxylé, un ester de sorbitan, un ester de polyglycérol, un ester de saccharose, un poloxamère, un polyglucoside d'alkyle, un heptaméthyltrisiloxane modifié par un oxyde de polyalkylène, un méthyléther d'allyloxypolyéthylène glycol et des mélanges de ceux-ci ;
le tensioactif amphotère est la lécithine ; et
le tensioactif cationique est choisi dans le groupe constitué par le bromure de cétyltriméthyl ammonium, le chlorure de cétyltriméthyl ammonium et des mélanges de ceux-ci.

10. Procédé selon la revendication 1, dans lequel le composé organique insoluble dans l'eau est sous une forme amorphe ou partiellement amorphe.

11. Procédé selon la revendication 1 comprenant, en outre, l'étape de cristallisation des nanoparticules, pour obtenir des nanoparticules organiques cristallines, dans lequel, de préférence, les nanoparticules sont cristallisées par vieillissement.

12. Poudre redispersable comprenant des nanoparticules d'un composé organique insoluble dans l'eau, les nanoparticules ayant un diamètre inférieur à 30 nanomètres et comprenant au moins 50 % en poids du composé organique insoluble dans l'eau, la poudre redispersable étant préparée par un procédé comprenant les étapes consistant à :

(i) préparer une microémulsion huile dans l'eau comprenant un composé organique insoluble dans l'eau, un solvant organique volatil non miscible à l'eau, de l'eau, et au moins un tensioactif, par :

(a) dissolution du composé organique insoluble dans l'eau dans le solvant organique volatil non miscible dans l'eau de manière à former une phase organique ; et
(b) mélange de la phase organique avec de l'eau et au moins un tensioactif de manière à former spontanément la microémulsion huile dans l'eau sans l'utilisation d'un homogénéisateur haute pression ou d'un instrument à cisaillement élevé ;

dans laquelle le diamètre des gouttelettes de microémulsion est inférieur à 30 nm ; et
(ii) éliminer le solvant organique volatil non miscible dans l'eau et l'eau de manière à former la poudre redispersable comprenant lesdites nanoparticules, dans laquelle lesdites nanoparticules sont sous une forme particulaire, le solvant organique volatil non miscible dans l'eau et l'eau étant éliminés simultanément.

13. Poudre redispersable selon la revendication 12, la poudre pouvant être redispersée dans l'eau pour former une dispersion aqueuse des nanoparticules.

14. Poudre redispersable selon la revendication 12, comprenant au moins 10 % en poids de nanoparticules d'un composé organique insoluble dans l'eau, les nanoparticules étant sous une forme particulaire.

15. Poudre redispersable selon la revendication 12,
dans laquelle le composé organique insoluble dans l'eau est choisi dans le groupe constitué par des agents pharmaceutiquement actifs, des agents actifs cosmétiques, des antioxydants, des conservateurs, des colorants, des additifs alimentaires, des composés actifs dans le domaine agricole, des réactifs utilisés dans des réactions chimiques et biochimiques et des parfums ; ou
dans laquelle le composé organique insoluble dans l'eau est sous une forme amorphe ou une forme partiellement amorphe ; ou

comprenant, en outre, au moins un polymère.

FIGURE 1

FIGURE 2

FIGURE 3

0.2 μm

FIGURE 4

## REFERENCES CITED IN THE DESCRIPTION

### Patent documents cited in the description

- US 5250236 A
- WO 2005072709 A
- WO 2005102507 A
- WO 2005020933 A
- WO 0188046 A
- US 5879715 A
- US 5874029 A
- US 20050170004 A
- US 5472706 A
- US 5750142 A
- EP 211257 A
- US 4534891 A
- US 5112688 A
- US 5145842 A

### Non-patent literature cited in the description

- **DEBUIGNE et al.** *Langmuir,* 2000, vol. 16 (20), 7605-7611
- **DEBUIGNE et al.** *J. Pharm Belg.,* 2000, vol. 55 (2), 59-60
- **TROTTA M. et al.** *Int. J. Pharm.,* 2003, vol. 245, 235-242
- **OZER et al.** *J. App. Poly. Sci.,* 2000, vol. 78 (3), 569-575
- **MAGDASSI ; BEN MOSHE.** *Langmuir,* 2003, vol. 19 (3), 939-942
- **DESGOUILLES et al.** *Langmuir,* 2003, vol. 19, 9504-9510
- **YONCHEVA et al.** *J. Microencapsul.,* 2003, vol. 20 (4), 449-458
- **HSU et al.** *AAPS PharmSciTech,* 2003, vol. 4 (3), E32
- **SASSON et al.** Insecticide Design Using Advanced Technologies. Springer-Verlag
- **FRIBERG et al.** Microemulsions Structure and Dynamics. CRC Press Inc, 1987
- **TAN ; PFISTER.** *Pharm Sci and Tech Today,* 1999, vol. 2, 60-69